# EUROPEAN PATENT APPLICATION

(11) **EP 1 852 186 A1**
(43) Date of publication of application: **07.11.2007**
(21) Application number: 06113457.3
(22) Date of filing: 03.05.2006
(51) Int. Cl.: B01L 3/00, C12Q 1/68, G01N 33/53

(54) **Method and means for multiparametric assays being compartmentalized**

(71) Applicant: Eppendorf Array Technologies, 5000 Namur (BE)
(72) Inventor: Mainfroid, Véronique, 4300 Waremme (BE); Koehn, Heinz, 22339 Hamburg (DE); Remacle, José, 5020 Malonne (BE)
(74) Representative: pronovem

(57) **Abstract**

The present invention is related to a method and means for detecting and/or quantifying multiple different targets in one assay by avoiding interferences between the some recognitions reagents (5, 6) necessary for performing the assay. The invention is also well adapted for the identification of the composition of a target when being composed of different subunits. The invention is also well suited for the identification of different targets bound to the same capture probe (4).

SEQUENCE LISTING

<110>  Eppendorf Array Technologies

<120>  Method and means for multiparametric assays being
       compartmentalized

<130>  BP.EATE.010B/EP

<160>  25

<170>  PatentIn version 3.3

<210>  1
<211>  7
<212>  DNA
<213>  Artificial Sequence

<220>
<223>  AP1 transcriptional factor family DNA binding motif (TRE)

<400>  1
tgastca                                                                 7


<210>  2
<211>  8
<212>  DNA
<213>  Artificial Sequence

<220>
<223>  ATF/CREB transcriptional factor family DNA binding motif (CRE)

<400>  2
tgacctca                                                                8


<210>  3
<211>  5
<212>  DNA
<213>  Artificial Sequence

<220>
<223>  C/EBP transcriptional factor family DNA binding motif (CCAAT box)

<400>  3
ccaat                                                                   5


<210>  4
<211>  6
<212>  DNA
<213>  Artificial Sequence

<220>
<223>  c-Myc transcriptional factor family DNA binding motif (E-box)

<400>  4
cacgtg                                                                  6


<210>  5
<211>  9
<212>  DNA
<213>  Artificial Sequence

<220>
<223>  Egr transcriptional factor family DNA binding motif (GC-rich
       sequence)

<400>  5
gcgggggcg                                                                9

<210>  6
<211>  8
<212>  DNA
<213>  Artificial Sequence

<220>
<223>  E2F/DP transcriptional factor family DNA binding motif

<400>  6
tttsscgc                                                                 8

<210>  7
<211>  4
<212>  DNA
<213>  Artificial Sequence

<220>
<223>  Ets transcriptional factor family DNA binding motif

<400>  7
ggaw                                                                     4

<210>  8
<211>  6
<212>  DNA
<213>  Artificial Sequence

<220>
<223>  GATA transcriptional factor family DNA binding motif

<400>  8
wgatar                                                                   6

<210>  9
<211>  13
<212>  DNA
<213>  Artificial Sequence

<220>
<223>  IRF transcriptional factor family DNA binding motif (ISRE)

<220>
<221>  misc_feature
<222>  (7)..(8)
<223>  n is a, c, g, or t

<400>  9
rrtttcnntt tcy                                                          13

<210>  10
<211>  8
<212>  DNA
<213>  Artificial Sequence

<220>
<223>  IRF transcriptional factor family DNA binding motif (GAS)

<220>

<221>  misc_feature
<222>  (3)..(3)
<223>  n is a, c, g, or t

<220>
<221>  misc_feature
<222>  (5)..(7)
<223>  n is a, c, g, or t

<400>  10
ttncnnna                                                                 8


<210>  11
<211>  6
<212>  DNA
<213>  Artificial Sequence

<220>
<223>  MRF transcriptional factor family DNA binding motif (E-box)


<220>
<221>  misc_feature
<222>  (3)..(4)
<223>  n is a, c, g, or t

<400>  11
canntg                                                                   6


<210>  12
<211>  6
<212>  DNA
<213>  Artificial Sequence

<220>
<223>  NFAT transcriptional factor family DNA binding motif

<400>  12
wggaaa                                                                   6


<210>  13
<211>  10
<212>  DNA
<213>  Artificial Sequence

<220>
<223>  NFkB/Rel transcriptional factor family DNA binding motif


<220>
<221>  misc_feature
<222>  (5)..(6)
<223>  n is a, c, g, or t

<400>  13
gggrnnyycc                                                              10


<210>  14
<211>  8
<212>  DNA
<213>  Artificial Sequence

<220>
<223>  Oct transcriptional factor family DNA binding motif (POU motif)

<400>  14
atgcaaat                                                                 8


<210>  15
<211>  10
<212>  DNA
<213>  Artificial Sequence

<220>
<223>  Hox transcriptional factor family DNA binding motif (Pbx/Hox
       motif)


<220>
<221>  misc_feature
<222>  (5)..(6)
<223>  n is a, c, g, or t

<400>  15
tgatnnatkr                                                              10


<210>  16
<211>  13
<212>  DNA
<213>  Artificial Sequence

<220>
<223>  PPAR transcriptional factor family DNA binding motif (DR-1)


<220>
<221>  misc_feature
<222>  (7)..(7)
<223>  n is a, c, g, or t

<400>  16
aggtcanagg tca                                                          13


<210>  17
<211>  14
<212>  DNA
<213>  Artificial Sequence

<220>
<223>  RAR/RXR transcriptional factor family DNA binding motif (RARE)


<220>
<221>  misc_feature
<222>  (7)..(8)
<223>  n is a, c, g, or t

<400>  17
aggtcannag gtca                                                         14


<210>  18
<211>  15
<212>  DNA
<213>  Artificial Sequence

<220>
<223>  RAR/RXR transcriptional factor family DNA binding motif (RARE)


<220>

<221>  misc_feature
<222>  (7)..(9)
<223>  n is a, c, g, or t

<400>  18
aggtcannna ggtca                                                        15


<210>  19
<211>  16
<212>  DNA
<213>  Artificial Sequence

<220>
<223>  RAR/RXR transcriptional factor family DNA binding motif (RARE)


<220>
<221>  misc_feature
<222>  (7)..(10)
<223>  n is a, c, g, or t

<400>  19
aggtcannnn aggtca                                                       16


<210>  20
<211>  17
<212>  DNA
<213>  Artificial Sequence

<220>
<223>  RAR/RXR transcriptional factor family DNA binding motif (RARE)


<220>
<221>  misc_feature
<222>  (7)..(11)
<223>  n is a, c, g, or t

<400>  20
aggtcannnn naggtca                                                      17


<210>  21
<211>  10
<212>  DNA
<213>  Artificial Sequence

<220>
<223>  Sp1-like transcriptional factor family DNA binding motif (GC box)

<400>  21
ggggcggggc                                                              10


<210>  22
<211>  6
<212>  DNA
<213>  Artificial Sequence

<220>
<223>  SREBP transcriptional factor family DNA binding motif (E-box)


<220>
<221>  misc_feature
<222>  (3)..(4)
<223>  n is a, c, g, or t

<400>  22
canntg                                                                  6


<210>  23
<211>  10
<212>  DNA
<213>  Artificial Sequence

<220>
<223>  SREBP transcriptional factor family DNA binding motif (SRE)

<400>  23
tcaccccact                                                              10


<210>  24
<211>  13
<212>  DNA
<213>  Artificial Sequence

<220>
<223>  STAT transcriptional factor family DNA binding motif (ISRE)


<220>
<221>  misc_feature
<222>  (7)..(8)
<223>  n is a, c, g, or t

<400>  24
rrtttcnntt tcy                                                          13


<210>  25
<211>  8
<212>  DNA
<213>  Artificial Sequence

<220>
<223>  STAT transcriptional factor family DNA binding motif (GAS)


<220>
<221>  misc_feature
<222>  (3)..(3)
<223>  n is a, c, g, or t

<220>
<221>  misc_feature
<222>  (5)..(7)
<223>  n is a, c, g, or t

<400>  25
ttncnnna                                                                8

## Description

### Field of the invention

The present invention is related to a method to avoid interferences between recognition reagents when performing an assay for detecting and/or quantifying multiple different targets in the assay. The method is also related to the identification of different targets which bind to the same capture probe.

The present invention is also related to a solid support for performing the method of the invention upon assays containing compartmentalized chambers.

The present invention is also related to a kit including this solid support.

### Background of the invention

Biological samples are difficult to analyze, because of the complexity of the molecules being present in biological organisms and the large number of molecules having close chemical or physical characteristics. On the other side, there is a need to perform analyses on materials as small as possible given the fact that in many analyses, the amount of material available is very limited. The assays for multiple components are now performed on a miniaturization scale using microarrays for the transcriptional analysis of the genes. This application takes advantage of the specific recognition of the double strands of DNA. However in the proteomic field, the recognition of specific proteins among the thousand ones present in a single cell is very difficult. The proteins are all constituted by 20 amino acids and their chemical or physical properties are very similar. One way to specifically recognize a protein is to use specific antibodies or specific molecules which target particular parts of the protein, such as epitopes, which are not present on other proteins. Miniaturized proteomic assays are best made by the use of proteins microarrays, where the proteins are immobilized on a support and recognized by specific reagents. However, when multiple proteins are present in a same sample and have to be detected simultaneously by combining multiple specific reagents, the process becomes difficult given the interference of the reagents mainly due to cross-reactions with other protein targets and the increase of the background signal.

The detection of proteins is best performed by indirect immuno-labeling methods typically comprising a multi-step process where the protein targets are first contacted with capture probes being on a solid support either a well of a multiwell plate or in the form of an array. However, primary antibodies, even being monoclonal or purified by affinity chromatography, can exhibit significant residual cross-reactivity with other proteins having similarities in structure or sequence with their target protein. The background problem is particularly apparent in complex samples such as in disease tissues, and reduces the usefulness and sensitivity of the assay. Thus, the simultaneous detection of more than one target in a sample without this significant background interference depends on the availability of primary antibodies that 1) do not cross-react with proteins intrinsic to the sample being examined, 2) recognize specifically their target 3) do not bind onto each other. This interference problem limits the number of targets which can be simultaneously detected in the same sample.

The detection of intracellular proteins which compose the signaling cascades is particularly concerned by those problems. They are present in complex biological samples, since cytoplasmic and total protein extracts prepared from i.e. mammalian cells are composed of a multitude of proteins with close physico-chemical properties. Contacting such samples with a solid support generates some background which is usually attributed to the non-specific binding of proteins onto the support, and their subsequent (specific) detection by the recognition reagents used in the assay, due to their residual cross-reactivity. This problem is classically limited by the use of blocking agents such as dried milk or bovine serum albumin, but determining the optimal blocking conditions in an assay always results from a compromise between the maximal reduction of the background signals and the minimal affectation of the specific signals. This compromise highly depends on, and thereby limits, the number of proteins that can be detected simultaneously in one assay, as increasing the number of recognition reagents which have to be combined for the detection step favors the background signals.

Some signaling proteins exhibit structural similarities, which renders their specific detection particularly challenging. Members of the growing family of Mitogen Activated Protein (MAP) kinases, which are related serine/threonine kinases, are a nice illustration. There are currently 11 vertebrate members identified, that are classified in 5 sub-families based on primary sequence homology. The simultaneous detection of several sub-family members in the same assay is difficult since the recognition reagents often cross-react with more than one MAP kinase member.

Many signaling proteins require post-translational modification(s), most frequently phosphorylation events, to become activated. Determining the activation status of a sample often requires to simultaneously quantify both forms, activated and non activated of several proteins of the activation pathways. This cannot be done on a same sample and in a same experiment, since mixing the detection reagents specific to both forms of a same protein would generate additive signals which cannot be subsequently decomposed to determine the contribution of each form.

The simultaneous detection of multiple transcriptional factors is also confronted to some technical limitations. Upon stimulation by intracellular signaling cascades, transcriptional factors become active and acquire the capacity to regulate the genes under their control through their binding to specific double-stranded DNA sequences in the promoter regions. Many transcriptional factors can bind DNA without being activated, however, and their transcriptional capacity then depends on post-translational events such as phosphorylation, tridimensional structure modification or cofactor binding. Examples of transcriptional factors whose activation involves a phosphorylation are listed in table 1. The detection of transcriptional factors is usually performed in assays monitoring their binding to specific DNA sequences. Therefore, the simultaneous detection of the non-activated and activated forms of a transcriptional factor from the above-mentioned category cannot be made in a same assay, as the corresponding recognition reagents cannot be used simultaneously.

In addition, many transcriptional factors recognize the same specific DNA sequences, and are therefore grouped into structural families (see Table 2). Examples are the STAT family of transcriptional factors, which contains the factors STAT1 to STAT6, the AP1 family, mainly composed of c-Fos, FosB, c-Jun, JunB and JunD, the NFκB family containing p50, p52, p65 and c-Rel and the CREB family, whose main representatives are CREB and ATF2. Here again, the simultaneous detection of more than one member of a same family is not possible in a same assay, as mixing the corresponding recognition reagents at the detection step is incompatible with their specific detection.

A characteristic common to many transcriptional factors is that they are transcriptionally active only as homo- or heterodimers. These dimers can involve proteins from a same family. As an example, AP1 can be composed of c-Fos and c-Jun, two c-Jun molecules or c-Jun and JunB, while c-Rel can heterodimerize with p50 or RelA to form NFκB complexes. Heterodimers can also involve proteins from different families. As examples, C/EBPα can dimerize with c-Fos, c-Jun can dimerize with CREB, and MyoD with E12. These and other examples are listed in Table 3. A same assay, where the hererodimeric transcriptional factor is immobilized on its specific DNA sequence, can only use one of the two recognition reagents which would be necessary to detect the individual monomers. The characteristics of transcriptional factors *per se* are therefore not compatible with the simultaneous detection of some of them.

### State of the art

The international Patent Application W00250537 provides a method for simultaneously detecting and quantifying multiple target molecules in a single reaction well of a reaction surface. Antibodies to different target molecules are bound to the surface. The target molecules form complexes with the bound antibodies. After a washing step, an enzyme-bound antibody mixture is added to the reaction surface. Each antibody has an affinity for a given target and carries a specific enzyme. The sequential addition of specific enzyme substrates permits the detection of multiple target molecules. The number of targets possibly detected is limited by the number of enzymes and substrates available.

The US application 20050069962A1 provides a method for detecting multiple targets in a sample by contacting the targets with a pooled subset of immuno-labeled complexes, each complex comprising a target binding antibody and a labeling reagent being a labeled monovalent protein. Each subset is distinguished from each other by the target-binding antibody or a ratio of label to labeling reagent or a ratio of labeling reagent to the target binding antibody or by a physical property of the label. The method requires the step of complexing labeled monovalent protein to target binding antibody prior to the addition with a biological sample.

The US Patent 5,763,158 provides a method for simultaneously testing a sample for the presence of multiple target antigens or antibodies. The sample is contacted with a plurality of different binding sites attached to a support, each binding site being composed of a ligand-enzyme complex in which the ligand is attached in proximity to the enzyme's active site such that the enzymatic activity of the ligand-enzyme complex is changed when the target antibody or antigen is present in the sample. The change of enzymatic activity has to be compared to a control value. The number of targets possibly detected is limited by the number of available ligand-enzyme complexes.

However, none of these documents addresses the particular problem of the simultaneous detection of multiple targets in complex sample mixtures and using different recognition reagents which can possibly cross-react with different targets or provide high background when combined.

### Aims of the invention

A first aim of the present invention is to provide a method and kit with improved selective detection and/or quantification of multiple targets in a miniaturized format and which does not present the drawbacks of the state of the art.

A preferred aim is to improve the specificity of an assay when simultaneously detecting multiple targets present in complex biological samples, to decrease the background generated in the assay, and to allow a discriminated identification between several targets which bind to or which cross-react with the same capture probe.

Another aim is to provide a method and kit for determining the composition of target when composed from different subunits.

### Summary of the invention

In assays performed on multiple targets in a complex biological sample interferences are often observed between the multiple reagents necessary to obtain a specific detection of the multiple targets.

The present invention proposes a solution to this problem and is related to a method and means for a detection and/or quantification of multiple and different targets in a sample by:
- Incubating a sample solution containing the targets into a chamber comprising at least two compartments, in the presence of capture probes specific of the targets and being immobilized on a solid support in the compartments (step 1);
- performing a detection and/or quantification of the targets bound to the capture probes by successive additions of solutions (v1, v2) containing reagents (step 2, 3, 4) and/or washing solutions (step 1', 2', 3', 4') into the chamber and/or in the compartments;
wherein in at least one step (step 2), a solution (v1) of recognition reagents specific of a first group of bound targets are incubated in a first compartment of the chamber and a second solution of recognition reagents specific of a second group of bound targets are incubated in a second compartment of the chamber, being not in fluidic contact with the first compartment of the same chamber, and wherein in at least another step (step 1, 3, 4) a solution (v2) is added to the chamber allowing a fluidic contact with all the compartments of the chamber.

The invention is also related to a solid support for a detection and/or quantification of multiple and different targets in a sample, this support comprising one or more chamber(s) having at least two separate compartments, and capture probes being immobilized on a solid support (surface) in the compartments and wherein the compartments are in fluidic contact or are not in fluidic contact with each other according to the volume (v1, v2) of solution added in the chamber.

The present invention is also related to a kit for a detection and/or quantification of multiple different targets in a sample, which comprises the solid support, according to the invention, recognition reagents being primary antibodies or specific hypervariable portions thereof and being specific of the different targets; optionally a universal reagent being (a) secondary labeled antibody (ies) directed against all the recognition reagents and optionally a labeling reagent being a specific substrate(s) of the universal reagent.

The invention also relates to an apparatus for the detection and quantification of the targets bound to the solid support, the apparatus comprising:
a detection and quantification device of a signal formed at the location of the binding between the target and the capture probe on the solid support (of the invention), preferably also a reading device of information recorded on a surface of said solid support, a computer program for recognizing the discrete regions bearing the bound target molecules upon their corresponding capture probes and their locations, preferably also a quantification program of the signal present at the locations and a program for correlating the presence of the signal at these locations with the diagnostic and the quantification of the targets to be detected according to the invention and assigning the locations of the targets according to the compartment to which they belong.

The present invention will be described in more details in the following examples, in reference to the enclosed figures presented as non-limiting illustrations of the various embodiments of the present invention.

### Short description of the figures and tables

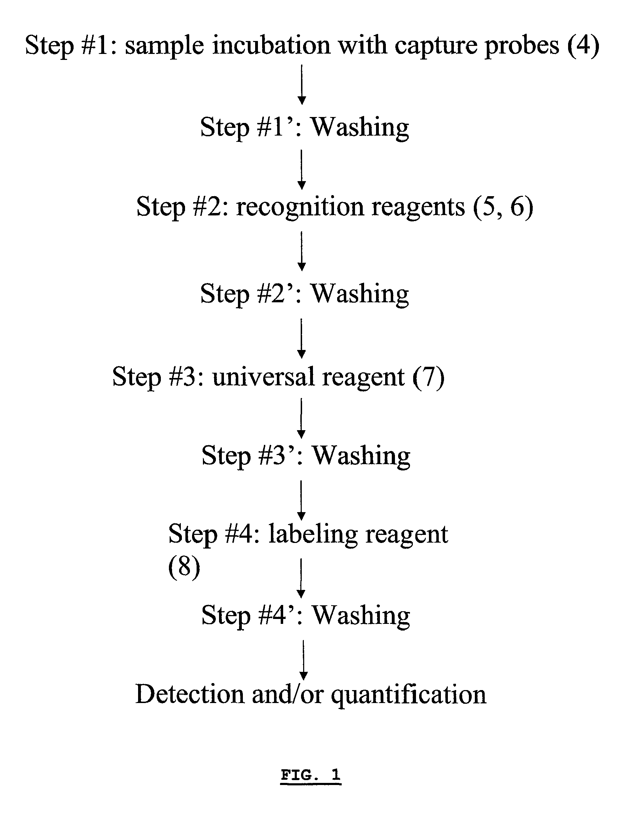

Figure 1 is a schematic representation of preferred steps required in the invention for the detection of multiple targets from the same sample: different steps (#1, #2, #3, #4) are performed either in a chamber with all the compartments being in fluidic contact with each other (steps #1, #3, #4) or in separated compartments being not in fluidic contact with each other (step #2). The steps #3 and/or #4 are optional and depend of the labeling method.

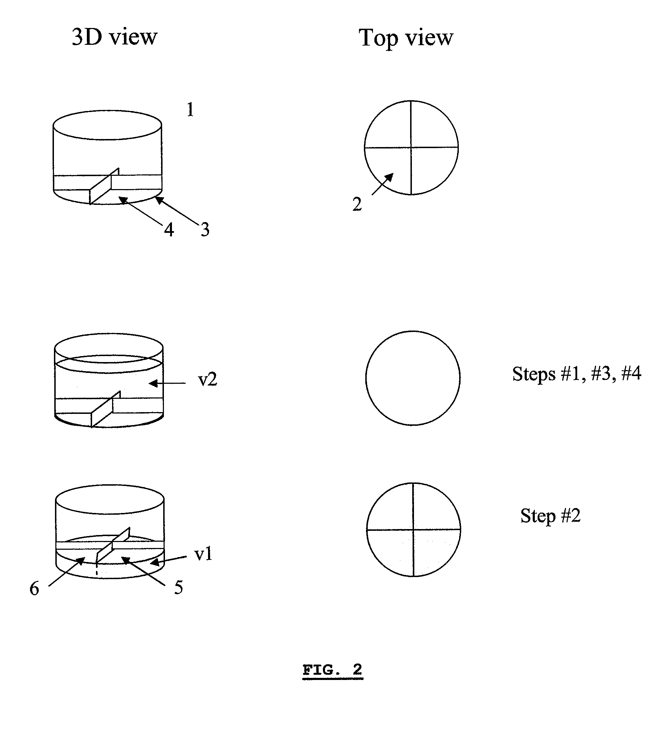

Figure 2 represents a 3D view and a top view of a preferred chamber (1) of the invention comprising a solid support (3) having fixed capture probes (4) and four compartments (2). The chamber is compatible with fluidic and non fluidic contacts between the different compartments depending on the volume of solution introduced into the chamber. Steps #1, #3 and #4 from figure 1 are preferably performed with a volume of solution (v2) allowing a fluidic contact between all the compartments of the chamber and step #2 with a smaller volume of solution (v1) allowing separate incubation of different recognition reagents (5, 6) in the different compartments.

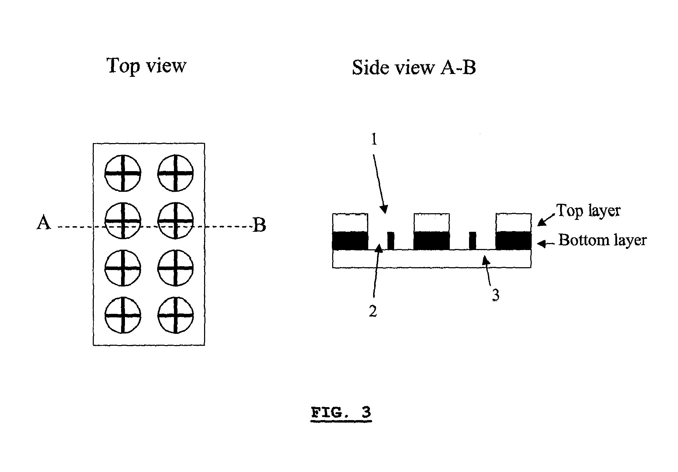

Figure 3 represents a top view and a side view (A-B) of a preferred device of the invention comprising a solid support (3) covered with 2 layers of materials (bottom and top layers) which are both cut in order to delimitate the chambers and one is partly cut (bottom layer) so that it delimitates the compartments inside each chamber. In this proposed device, the solid support (slide) contains 8 chambers (1), each having 4 compartments (2).

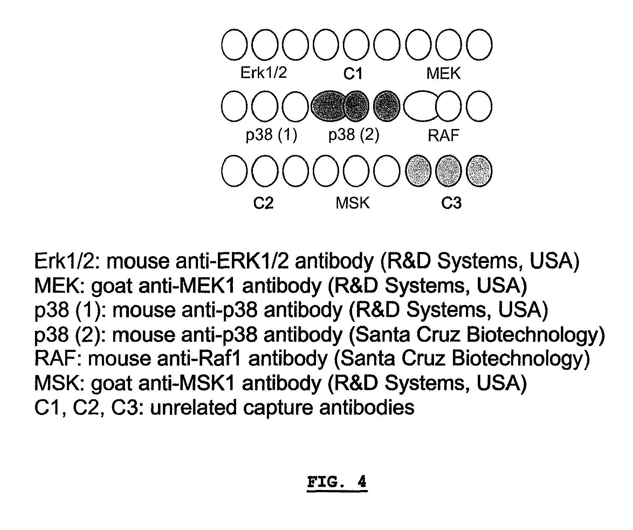

Figure 4 shows the different antibodies used as capture probes in protein array. Results are given in examples 1 and 2.

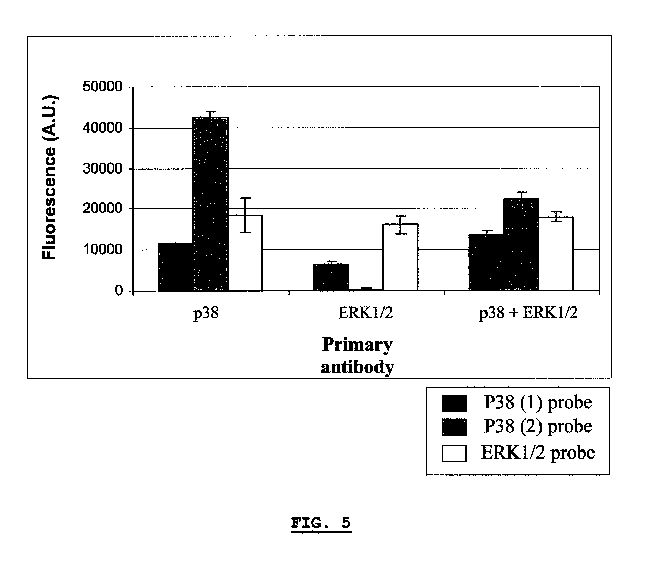

Figure 5 presents the fluorescence signals obtained for the detection of two proteins (p38 and ERK1/2) present in the same sample on capture probes being specific antibodies. Their respective recognition reagents ( primary antibodies) are added in the same compartment (p38 + ERK1/2) or in separate compartments (p38 alone) and (ERK1/2 alone).

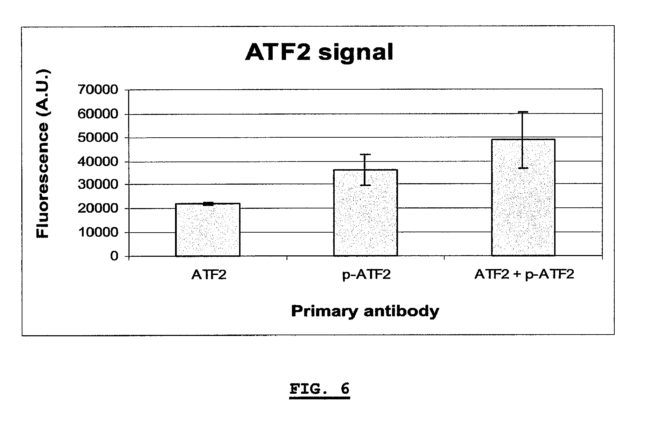

Figure 6 presents the fluorescence signals obtained on a TFChip MAPK array (Eppendorf, Germany) for the detection of the non-activated and the activated forms of a same transcriptional factor (ATF2) present in a sample. The respective recognition reagents ( primary antibodies ) are added in the same compartment (ATF2 + p-ATF2) or in separate compartments (ATF2 alone) and (p-ATF2 alone.

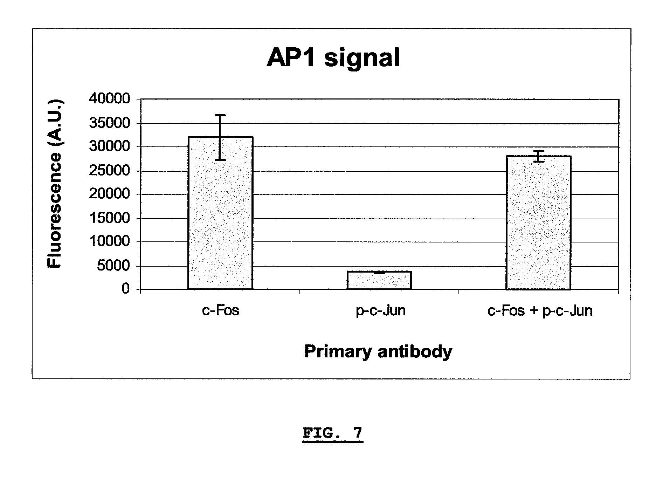

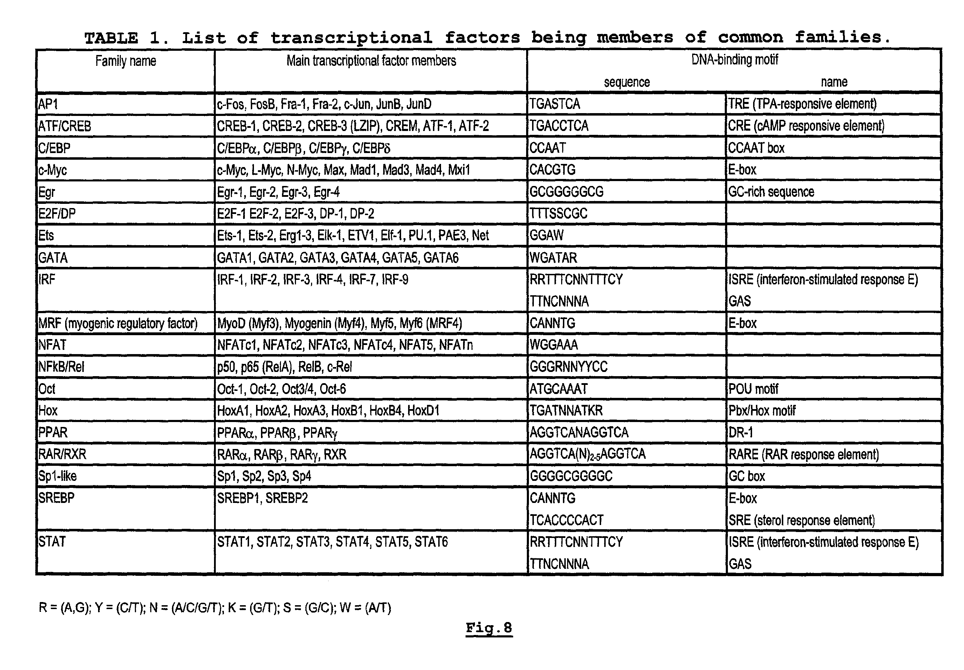

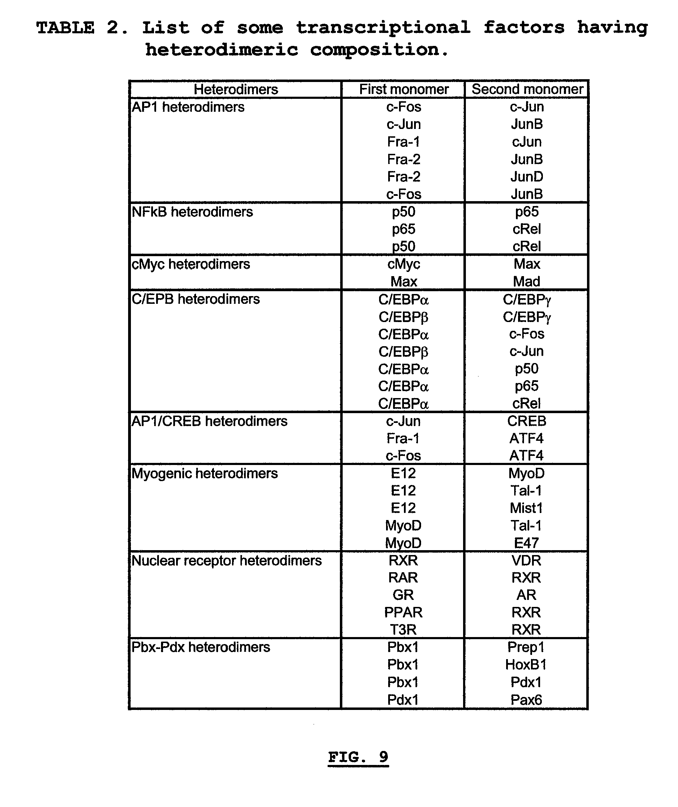

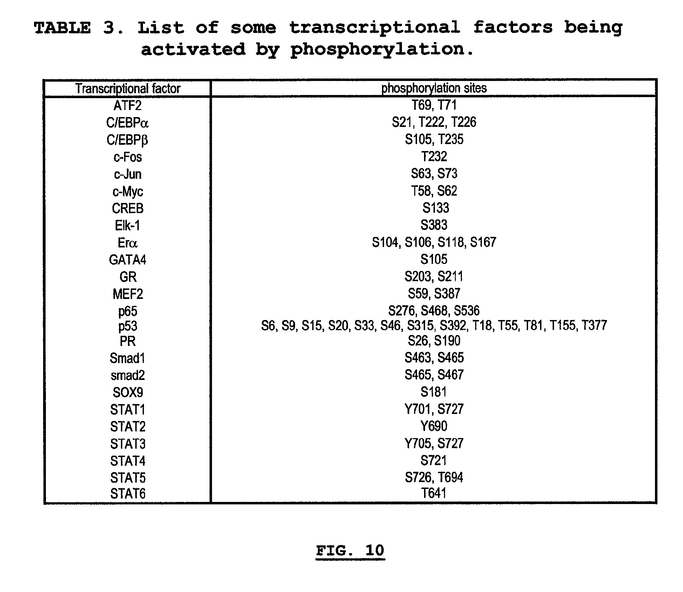

Figure 7 presents the fluorescence signals obtained on a TFChip MAPK array (Eppendorf, Germany) for the detection of two monomers of the AP1 transcriptional factor dimer. The respective recognition reagents are added in the same compartment (c-Fos + p-c-Jun) or in separate compartments (c-Fos alone) and (p-c-Jun alone).

Table 1 lists transcriptional factor families and their main members, as well as the DNA-binding motif to which they bind.

Table 2 lists examples of heterodimeric transcriptional factors and their monomer constituents.

Table 3 lists transcriptional factors whose phosphorylation modifies their transcriptional activity, and their phosphorylation sites.

### Definitions

Unless otherwise defined, all technical and scientific terms used herein have the same meaning as commonly understood by one person ordinary skilled in the art to which this invention belongs.

According to the invention, the term 'protein' refers to a complete protein or a fragment thereof, such as polypeptide, oligopeptide, etc. Fragment means portion of at least 5 consecutive amino-acid residues, preferably at least 25 consecutive amino-acid residues, more preferably at least 50 amino-acid residues.

"Transcriptional factors" are proteins which bind to specific sequences of double-stranded DNA and modulate, either activate or repress, the transcription of the DNA. They are composed of a DNA binding domain and a transactivating domain responsible for their activity and they are part of a large protein complex which interacts with the transcriptional machinery for regulating its activity. Transcriptional factors are activated through different mechanisms such as translocations, post-translational modifications like phosphorylations, and association/dissociation with cofactors, such as co-activators and co-repressors, which respectively increase and decrease the transcriptional regulation. The activity of a transcriptional factor refers to its capacity to modulate or regulate the transcription of DNA. The DNA binding capacity of a transcriptional factor refers to its capacity to bind a specific sequence of double-stranded DNA.

"Families of transcriptional factors" as understood in the present invention are composed of transcriptional factors which bind to the same DNA sequences.

The terms "post-translational modification" include phosphorylation, acetylation, glycosylation, lipid attachment, methylation, disulfide bond formation and proteolytic cleavage.

"Protein phosphorylation" refers to post-translational protein phosphorylation. "Phosphorylation" includes phosphorylation on a protein residue being a tyrosine, serine, threonine and/or histidine. Antibodies that can be used to detect these modifications include phosphotyrosine-specific antibody, phosphoserine-specific antibody, and phospho-threonine-proline antibody, for example. Antibodies that may be used to detect these modifications also include antibodies specific to (a) phosphorylated residue(s) of a protein, or a fragment of the protein containing the phosphorylated residue(s).

As used herein, "antibody" includes immunoglobulins which are composed of two light chains and two heavy chains linked by disulfide bounds and also hypervariable portions fragments, such as Fab, (Fab)2, Fv or single chain antibody variable fragments (scFv).

"Binding partner of a known protein" refers to any molecule that can physically interact, directly or indirectly, with a known protein. If the known protein is a transcriptional factor, the interaction with the protein partner can result in the transcriptional factor activation or its DNA binding to a specific sequence. Examples of binding partners of transcriptional factors are co-activators and co-repressors.

The terms "sample" include biological samples, which are biological fluid or a biological tissue or an extract or fraction thereof. Examples of biological fluids include urine, blood, plasma, serum, saliva, semen, stool, sputum, cerebral spinal fluid, tears, mucus, amniotic fluid, cell cultured or the like. Biological tissues are aggregates of cells, usually of a particular kind together with their intercellular substance that form one of the structural materials of a human, animal, plant, bacterial, fungal or viral structure, including connective, epithelium, muscle and nerve tissues. Examples of biological tissues also include organs, tumors, lymph nodes, arteries and individual cell(s). Biological samples also include in vitro cultured cells, extracts or fractions thereof. The in vitro cultured cells include transformed cell lines or primary cells. These cells can be treated, wherein the treatment is physical, chemical and physiological or a drug administration. 'Sample' also encompasses proteins, nucleotide sequences, polysaccharides, lipids, or a mix thereof.

As used herein, "capture probes" refer to molecules, complexes or combination of different or similar molecules, that are capable of specifically binding to a "target" (or target molecule) being a polynucleotide, a protein, a family of proteins, a polysaccharide or to one or more member (s) of a plurality of target proteins, or portion(s) thereof. The Polynucleotide capture probes bind to other polynucleotides through base pairing. The protein capture probes are peptides, proteins, e.g., antibodies or hypervariable portions thereof containing various parts of the heavy and/or the light chains of the antibodies, scaffold proteins or receptors or even polynucleotides e.g. aptamers being able to specifically recognize proteins. Receptors refer to molecules that have an affinity for a given ligand. Receptors may be naturally occurring or synthetic molecules.

Capture probes for transcriptional factors also include nucleotide sequences to which the transcriptional factors bind. They preferably comprise double-stranded DNA sequences containing a site (a specific sequence portion) for the specific binding with transcriptional factors or other DNA-binding proteins. These sequences may be naturally occurring or genetically engineered DNA sequences. The specific binding sites for the transcriptional factors or DNA-binding proteins may be naturally occurring sites (sequences) or consensus binding sites (sequences).

The term "single capture probe species" is a composition of related polynucleotides or polypeptides for the detection of a given sequence by base pairing hybridization or by molecular recognition between polypeptides or proteins or polynucleotides. Polynucleotides or polypeptides are synthesized either chemically or enzymatically or purified from samples but the synthesis or purification is not always perfect and the capture probes can be contaminated by other related molecules like shorter polynucleotides or other polypeptides. The essential characteristic of a capture species for the invention is that the capture probe species can be used to capture a given target being another polynucleotide or polypeptide sequence or polysaccharide.

The term `solid support' refers to any material made of elements selected from the group consisting of polymers, glass, metals or silicium; said material (surface) being able to fix directly or be covered by a layer (surface) able to fix the capture probes necessary for the targets interaction and assay. The forms of the supports are preferentially, but not limited to, multi-well plates of 96, 384 or 1536 wells, micro-arrays with planar surfaces or with cavities, where the different capture probes are present in different spots on the micro-arrays, or (possibly magnetic) microbeads for using for their assay in FACS machines or for facilitating their washing through their magnetic properties.

"Micro-arrays and arrays" mean supports on which capture probes are immobilized at specific locations in order to be able to bind to the given specific targets. The most common arrays are composed of single capture probes species being present in some locations of a support being or not a substrate for their binding. The array is preferentially composed of spots of capture probes deposited at a given location on the surface or within the support or on the substrate covering the support. The capture probes are present on the support in various forms being but not limited to spots. One particular form of application of array is the presence of capture probes in wells having either one of several different capture probes per well and being part of the same support. In one particular application of this invention, arrays of capture probes are also provided on different supports as long as the different supports contain specific capture probes and may be distinguished from each other in order to be able to quantify the specific targets. This can be achieved by using a mixture of beads having particular features and being able to be recognized from each other in order to quantify the bound targets.

The term "recognition reagents" means molecules either chemically or biologically synthesized which are able to specifically recognize and bind specifically to a given target (such as a polynucleotide, a protein, a family of proteins, or a polysaccharide). Family of proteins means proteins having at least a common feature such as but not limited to a common structural domain or epitope. Preferably, recognition reagents are antibodies (monoclonal or polyclonal) or part of antibodies such as proteins having the hypervariable portion of the antibodies. Recognition reagents are also chemical products being part of a library of molecules and having different properties so that they can recognize different features of the targets and be used as recognition molecules for different targets. "Recognition reagents" have similar properties as the capture probes since they can recognize the presence of the target and lead to its detection through direct of indirect labeling.

The term "universal reagent" means a reagent or a combination of reagents which specifically recognize and bind to all the recognition reagents used in the assay. The universal reagent reacts with all molecules of the same family, and is preferably an antibody directed against a family of molecules such as an anti-IgG of one species. In the present invention, the universal reagent normally specifically recognizes and binds to a common feature of all the recognition reagents used in the assay. Such common feature is usually a common structural domain or epitope, an incorporated hapten or biotin. There is usually only one universal reagent used in one assay but the use of a combination of reagents composing the universal reagent is also possible. As an example, a goat-anti-rabbit secondary antibody can be combined with a goat-anti-mouse secondary antibody to form the universal reagent which specifically recognizes and binds to recognition reagents composed of rabbit and mouse primary antibodies. The universal reagent is preferably labeled with a molecule which is either directly detected (i.e. fluorochrome such as cy3, cy5 or cy7) or indirectly after a chemical or enzymatic reaction (i.e. enzyme such as peroxidase or alkaline phosphatase or gold particle) .

The term "labeling reagent" is the reagent necessary to obtain a detectable signal from a label. Examples of labeling reagent are the substrates for enzymatic reaction leading to a detectable product being colored, chemiluminescent, bioluminescent or insoluble. Other examples of labeling reagent are the substrates (i.e. Ag+ and reducing agent) for chemical reaction such as metallic precipitation (i.e. silver deposit) catalyzed by a gold nanoparticle.

The terms "nucleic acid, bind substantially, hybridizing specifically to, background, quantifying" are as described in WO97/27317 which is incorporated herein by way of reference.

### Detailed description of the invention

The method is best used for the selective detection and/or quantification of multiple different targets in a miniaturized format by an incubation step of a biological sample (or an extract or a derivative thereof), possibly containing said targets, in the presence of capture probes (4) being immobilized on a solid support (3) surface having a chamber (1) comprising at least two compartments (2) .

In a preferred embodiment, the chamber (1) is composed of different compartments (2) which are in fluidic contact or are not in fluidic contact with each other according to the volume (v1, v2) of solution added in the chamber (1) .

For performing a detection and/or quantification of these targets bound to the capture probes (4), the chamber is best organized into compartments (2) by (possibly removable or openable) physical separations (9) which are limited in height (these compartment have a lower height than the one of the chamber (1) so that a fluidic contact between the compartments (2) is obtained, once a solution introduced in the compartments reaches a certain volume (v2) or higher or once the physical separations are removed or are open). The minimal volume for which all the compartments (2) of the chamber (1) are in fluidic contact is the critical fluidic volume (v2) . The compartment volume (v1) is the volume for which a liquid is present in a compartment alone and wherein there is no fluidic contact with another compartment and that does not contaminate the other compartment.
In another preferred embodiment, wherein the volume (v2) of solution added in the chamber is between 1.5 and 10 times the volume (v1) of solution added in each compartment multiplied by the number of compartments.

In another preferred embodiment, the volume (v1) of solution added in the compartment (2) is comprised between about 1 and about 20 µl and the volume (v2) of solution added in the chamber (1) is comprised between about 10 and about 1000 µl.

In a preferred embodiment, the steps (step 1, 3, 4) comprising addition of a volume (v2) of solution to the chamber (1) with fluidic contact between all the compartments (2) is a step selected from the group consisting of:
- incubation of a solution containing the targets with the capture probes (step 1),
- incubation with an universal reagent (7) being specific of the recognition reagents (5, 6) (step 3),
- incubation with labeling reagent (8) being specific of the universal reagent (7) (step 4).
- one or more washings steps (step 1', 2', 3' and/or 4') of the chamber.

In a preferred embodiment, the targets are selected from the group consisting of nucleotide sequences, peptides, proteins, polysaccharides, or a mixture thereof.

In another embodiment, the capture probes are selected from the group consisting of: polynucleotide sequences, peptides, proteins, antibodies or hypervariable portions thereof, polysaccharides, or a mixture thereof.

In further embodiment, the polynucleotides capture probes present in different compartments (2) allow for the detection and/or quantification of targets differing by a single nucleotide or the detection and/or quantification of different alternative splicing variants.

Preferably, the targets are proteins and the capture probes (4) are polynucleotide sequences, more particularly aptamers.

In another embodiment, the targets are proteins being preferably transcriptional factors and the capture probes (4) are composed of or contain polynucleotide sequences (preferably double-stranded DNA sequences) able to bind different transcriptional factors.

In a preferred embodiment, the method allows for the determination of activated transcriptional factors.

In an alternative embodiment, the targets are proteins being bound to transcriptional factors, being preferably phosphorylated as provided in table 3.

In still another embodiment, the targets are binding partners of a known protein being immobilized in different compartments (2).

In a preferred embodiment, the capture probes (4) are bound onto the solid support (3) in discrete regions in the form of an array having a density of at least 4 discrete regions per cm² and better 10 and even better 100 and even 1000.

In another embodiment, the array comprises capture probes (4) which are specific of different targets.

In an alternative embodiment, the (at least) two compartments (2) of the chamber (1) contain the same capture probes (4). Particularly, the same capture probes (4) are bound by different targets to de detected and/or quantified.

In a preferred embodiment, different capture probes (4) are immobilized in the different compartments (2) of the chamber (1).

In a specific embodiment, at least two compartments (2) of the chamber (1) contain one or even more than 4 or even more than 10 identical capture probes (4).

In still a preferred embodiment, the interference of cross reaction, or/and background is reduced significantly as provided by the present invention compared to non compartmentalized assays.

The method is well suited for the reduction of the complexity of the reagents which are used in at least one step of the detection method. Preferably the capture probes (4) present in the different compartments (2) are able to bind to least 10 and better 20 and still better 100 or even 1000 or more targets present in the same sample and which require the use of at least 10 or 20 or even 100 or even 1000 or more specific recognition reagents (5, 6) by the use of at least 2 and better 4 and still better 16 and still better 64 and even 384 or 1536 different compartments (2) .

In a particular embodiment, the method allows for the detection and discrimination and/or quantification of different targets being bound to the same capture probes (4). Particularly, the capture probes (4) are double-stranded DNA sequences comprising a specific sequence able to bind different targets being different transcriptional factors of the same family. The different targets are specifically detected in different compartments (2) using different recognition reagents (5, 6).

In a preferred embodiment, the recognition reagents (5, 6) are antibodies specific of the different targets.

Preferably, the recognition reagents (5, 6) carry a first member of a binding pair and the universal reagent (7) a second member of the binding pair.

In a preferred embodiment, the binding pair is selected from the group consisting of biotin/streptavidin, biotin/anti-biotin, hapten/receptor and antigen/antibody.

In a preferred embodiment, the universal reagent (7) is labeled and the label is directly detected or detected through the use of a labeling reagent (8).

Labels can be part of but are not limited to fluorochromes such as Cy3 or Cy5 or Cy7, enzymes such alkaline phosphatase, peroxidase and luciferase. In another embodiment, the label is gold and the labeling reagent (8) is Ag+ and a reducing agent.

The method is best performed using a universal reagent (7) which allows the detection of targets preferably labeled with non radioactive markers allowing a detection preferably by colorimetry, fluorescence, bioluminescence, electroluminescence or precipitation of a metal deposit (such as silver staining as proposed by EP1179180).. Said non radioactive test is preferably based upon a colorimetric assay resulting from a catalytic activity such as enzymatic or chemical reduction of silver cation. If direct method such as mass spectrum analysis is sensitive enough it can be used for direct detection of the bound factor.

In a preferred embodiment, the bound targets are best detected and quantified using the methods of colorimetry by absorption or transmission, light-scattering, fluorescence, bioluminescence, chemoluminescence, electroluminescence, electric, capacitance, internal reflection of light provided by evanescent waves, piezoelectric vibrations or the detection method for a metal precipitate such as the silver staining as proposed by EP1179180. If direct method such as mass spectrum analysis is sensitive enough it can be used for direct detection of the bound targets.

In a preferred embodiment, the signal associated with a capture probe (4) on the array(s) is quantified. The preferred method is the scanning of the array(s) with a scanner being preferentially a laser confocal scanner such as "ScanArray" (Packard, USA) for the detection of fluorescent labeled targets. The preferred detection and/or quantification scanner for colorimetric analysis is performed with the Silverquant scanner (Eppendorf, Germany). The resolution of the image is comprised between 1 and 500 µm and preferably between 5 and 50 µm. Preferably the array is scanned at different photomultiplier tube (PMT) settings in order to maximize the dynamic range and the data processed for quantification and corrections with the appropriated controls and standards (de Longueville et al., Biochem. Pharmacol. 64 , 2002,137-49).

Preferably the assay of targets being transcriptional factors is performed according to US 2004/0185497 and US 2003/0162211. The detection of transcriptional factors or proteins able to bind DNA sequences and present in cell lysate (including nuclear lysate) or present in a body fluid has to be very sensitive with less than about 10⁻¹² mole of transcriptional factor to be detected in 10 to 200 micro-liters in an assay and better having a limit of detection lower than 10⁻¹⁴ mole in the assay. Sensitivity of the transcriptional factor assay has to be sufficient for the detection of transcriptional factors naturally present in cell lysates.

According to a preferred embodiment, binding of the transcriptional factors (targets) to their specific double-stranded DNA sequences (capture probes) fixed on the solid support of the compartments is followed by incubation with labeled recognition reagents (5, 6) able to react with said transcriptional factors, preferably labeled antibodies or hypervariable portions thereof. Alternatively, the recognition reagents (5, 6), preferably antibodies, are not labeled and are then recognized by (a) universal reagent(s) (7) being preferably antibodies directed either against the recognition reagents or against a hapten present on the recognition reagents being preferably biotin. In another embodiment of the method, universal reagent (7) is another secondary binding protein, preferably protein A or protein G, which binds to antibodies.

The arrays or similar technology allow the simultaneous detection of different transcriptional factors present in the same sample, with all transcriptional factors binding to their specific double-stranded DNA sequence in the same reacting conditions. Solutions are available for example from Promega (Madison, USA) for the binding of several factors (Catalog E3581). Conditions which allow the binding of the factors studied may be optimized by the person skilled in the art.

In particular, the detection and/or the quantification of the different (activated) transcriptional factors are performed by the steps of:
a) obtaining (providing) a cell extract containing (activated) transcriptional factors;
b) contacting the cell extract with the support under conditions allowing the binding of (activated) transcriptional factors with a microarray made of at least 5 capture probes/cm² of the solid support surface, wherein each different capture probe is localized in a predetermined location of the said solid support surface, wherein each probe is specific of an (activated) transcriptional factor, and
c) detecting and/or quantifying signal(s) resulting from the binding of the (activated) transcriptional factors to their corresponding capture probes, and wherein the location of the signal(s) is related to the transcriptional factor identity present in said location.

Preferably, the capture probes for the transcriptional factor assay are composed of or contain double-stranded DNA sequences immobilized on the solid support surface at a concentration of at least 0.01 micromoles/cm², wherein these double-stranded DNA sequences comprise a specific sequence able to bind specifically an activated transcriptional factor and wherein the double-stranded DNA sequences are linked to the solid support surface by a spacer having a length of at least 6.8 nm.

More preferably, these double-stranded DNA sequences are linked to the support (surface) by a spacer of at least about 13.5 nm.

In another preferred embodiment, the capture probes are antibodies bound to the support (surface) and the detection of the bound transcriptional factors is then performed as classical protein detection well known by the person skilled in the art.

The method is well adapted for the detection of multiple proteins being present in a sample. In a preferred application, the sample containing multiple proteins to be detected is incubated with the compartmentalized support bearing capture probes being antibodies or fragments thereof or being able to capture proteins arranged in the form of array with spots being 100 to 400 µm in diameter. After washing, specific recognition reagents being preferably antibodies or related molecules, biotin labeled, are incubated in the different compartments so as to make them react with their target. The compartments are washed and anti-biotin antibodies labeled with nanogold particles are incubated in the chamber and after washing, a Silverquant reagent (Eppendorf Germany) is added in order to produce silver precipitation in the form of crystals which are then detected easily with a colorimetric scanner as the Silverquant scanner (Eppendorf, Germany). The digitalized image is then analyzed in order to determine the spots mean intensities and the data are treated in order to transfer the data of the spots into the protein content of the original sample.

The method is advantageously well adapted for screening for protein partners in transduction cascades using antibody microarrays. The method allows for the identification of binding partners of a known protein by combining the immunoprecipitation of the known protein by specific antibodies with the powerful specific and sensitive screening of partner molecules possibly bound to the known protein on microarrays. The detection of the partner molecules is preferably performed after separation of the known protein/partner complex using specific antibodies against the known protein. The bound partner is preferably co-immunoprecipitated with the known protein or separated on antibodies bearing beads and after separation of the complex, the partners will be specifically detected on the array. Since the number of possible partners to be screened is high, the present method is particularly well suited for an assay containing separate compartments each having a limited number of capture probes allowing to identify for possible bound partners on these capture probes; the method will provide the information of which partner peptides/proteins bind to a given protein in a given biological situation.

Polynucleotides are best detected taking advantage of the base pairing between the Adenine and the Thymine and between the Guanine and the Cytosine. The capture probes being immobilized are single strand DNA at least for part of it and the targets are polynucleotides which will hybridize on their complementary capture strand or having at least in part some complementary stretch of sequence. Preferably the capture polynucleotides bear a free amino group and are immobilized on an aldehyde bearing support as described in EP1313677 by spotting in the form of an array. The hybridization, labeling, detection and quantification is performed as described in the US10/439,767 and EP1266034

Carbohydrate or polysaccharide arrays can be used for the characterization of carbodydrate-protein or carbodydrate-carbohydrate interactions. The development of a carbochip must satisfy several requirements in order to provide selectivity and quantitative performance. First, the chip must prevent the non-specific adsorption of proteins from a contacting solution. Second, carbodydrate must be presented in a regular and homogeneous environment so that all immobilized ligands have equal activity towards soluble proteins and enzymes. Third, the density of carbohydrate ligands must be controlled because many carbohydrate-protein and carbohydrate-carbohydrate interactions are polyvalent in nature. Houseman and Mrksich have proposed an approach that meets these requirements. Polysaccharide arrays were prepared by the Diels-Alder-mediated immobilization of carbohydrate-cyclopentadiene conjugates to self-assembled monolayers that present benzoquinone and penta(ethylene glycol) groups. Polysaccharide arrays presenting ten monosaccharides have been used for profiling the binding specificities of several lectins (Houseman and Mrksich 2002, Chem. Biol., 9, 443-54).

The method is well suited for the reduction of the background level of an assay. In a preferred embodiment, the level of the background is reduced by a factor of 2 and still better by a factor of 4 compared to the corresponding non compartmentalized assay.

The method is well suited for the detection of targets which recognize the same capture probe(s) in different compartments (2). In a preferred embodiment, the method allows detecting and/or quantifying transcriptional factors targets which are different members from (a) same family (ies) and is best performed by:
(i) providing a solid support (3) having a chamber (1) with as many compartments (2) as family members to detect, and identical capture probes (4) containing the specific sequences for the transcriptional factor targets immobilized in each compartment,
(ii) incubating the sample possibly containing the transcriptional factor targets in the presence of the capture probes (4) immobilized in the different compartments (2) being in fluidic contact with each other (step #1),
(iii) incubating each compartment (2) separately with a different solution containing a set of recognition reagents (5, 6) which are specific to one transcriptional factor member of each family, the compartments (2) being not in fluidic contact with each other and the volume (v1) of the solutions being equal to the compartment volume (step #2),
(iv) incubating all compartments (2) of the chamber (1) with a solution (v2) being in fluidic contact with all compartments and containing a universal labeled reagent (7) specific for all the recognition reagents (5, 6) (step #3) and appropriate washing steps, and
(v) detecting and/or quantifying signals.

The method is particularly suitable for the identification of transcriptional factors of the AP1, STAT and NFκB families. Example of transcriptional factors families whose members can be detected and/or quantified with the method are presented in table 1 but are not limited to the content of table 1.

The method is also well suited for the identification of the different subunits or monomers of heterodimeric or multimeric proteins. The determination of the target composition is performed by using different specific recognition reagents (5, 6) in different compartments (2), preferably specific antibodies, which question the possible subunit or monomer composition of the immobilized target. The method is best performed by:
(i) providing a solid support (3) having a chamber (1) with two compartments (2), and having immobilized in each compartment capture probes (4) specific for the targets,
(ii) incubating the sample possibly containing the targets in the presence of the capture probes (4) immobilized in the different compartments (2) being in fluidic contact with each other (step #1),
(iii) incubating the first compartment with a solution (v1) containing recognition reagents (5) which are specific for the first monomers of the heterodimeric proteins, and the second compartment with another solution containing recognition reagents (6) which are specific for the second monomers of the heterodimeric proteins, the compartments being not in fluidic contact with each other,
(iv) incubating the two compartments (2) of the chamber (1) with a same solution (v2) being in fluidic contact with all compartments (2), and containing a universal labeled reagent (7) specific for all the recognition reagents (5, 6) (step #3), and appropriate washing steps, and
(v) detecting and/or quantifying signals.

An application is the determination of the composition of heterodimeric transcriptional factors, such as but not limited to those listed in table 2.

The method is also well adapted to detect the different subunits of a multimeric protein, and is best performed as described above, with the number of compartments equal to the number of subunits to be detected.

The method also preferably comprises the step of identification of at least one characteristic specific of a given target being a protein or/and an activated transcriptional factor. Some transcriptional factors bind to their specific DNA sequence without activating the transcriptional machinery. The activation is then associated with one or several specific changes, the most common one being the phosphorylation (or dephosphorylation) of specific residue(s) of the protein. An example of such transcriptional factor is CREB, which is only active when phosphorylated. Other examples are listed in table 3. The method allowing to detect and/or quantify targets which are the non-activated and activated forms of transcriptional factors is best performed by:
(i) providing a solid support (3) having a chamber (1) with two compartments (2), and having immobilized in each compartment (2) capture probes (4) containing the specific sequences for the transcriptional factor targets,
(ii) incubating the sample possibly containing the transcriptional factor targets in the presence of the capture probes (4) immobilized in the different compartments (2) being in fluidic contact with each other (step #1),
(iii) incubating the first compartment (2) with a solution containing recognition reagents (5) which are specific for the non-activated transcriptional factor forms, and the second compartment (2) with another solution containing recognition reagents (6) which are specific for the activated transcriptional factor forms, the compartments being not in fluidic contact with each other and the volume (v1) of the solutions being equal to the compartment volume (step #2),
(iv) incubating the two compartments (2) of the chamber (1) with a same solution (v2) being in fluidic contact with all compartments, and containing a universal labeled reagent (7) specific for all the recognition reagents (5, 6) (step #3), and appropriate washing steps, and
(v) detecting and/or quantifying signals.

More generally, the method of the invention can differentiate the DNA-binding capacity of a transcriptional factor from its activity. This activity is associated with one or several particular characteristics of the factor, the most common ones being the phosphorylation of specific residues, the association with co-activators or the dissociation from co-repressors. By using appropriate antibodies directed against these elements, it is possible to determine the amount of transcriptional factor in its active form.

In a particular embodiment, the method includes the assay for the activity of kinases, preferably the MAP kinases, involved in cell regulation or activation pathways. Activities of the kinases being part of the MAP kinase activation pathways are determined by measurement of the level of phosphorylation of the proteins, enzymes being the substrates of the kinases and phosphatases being part of the pathways or of synthetic peptides in an in vitro assay. The activity level of an enzyme is obtained by calculating the ratio between the level of specific phosphorylation to non-phosphorylated form or to the total enzyme content. The degree of activation of the enzymes is obtained by quantifying, in different compartments of the same chamber, preferably in the form of arrays, the amount of phosphorylated enzymes compared to the total amount of enzymes, both compartments being incubated with the same cell extract.

Quantitative determination of the different activated MAP kinases is preferably performed by the steps of:
(i) obtaining (providing) a cell extract containing activated MAP kinases and phosphatases,
(ii) determining the equilibrium between the activities of kinase/phosphatase enzymes on proteins participating in signal transduction into cells or quantitatively determining the level of phosphorylation of cellular proteins participating in signal transduction belonging to a cascade of phosphorylation leading to the activation of at least one transcriptional factor.

In a preferred embodiment of the invention, the assay for the kinase activity is realized according to the method described in US 2004/0265938.

In another embodiment of the invention, the assay for different kinase cascades is performed by detecting and/or quantifying the phosphorylation of the resulting phosphoproteins. Protein phosphorylation occurs on an amino acid residue selected from the group consisting of tyrosine, serine, threonine and histidine.

In still another embodiment, the activities of specific kinase/phosphatase enzymes are determined, which give an overview of the cell's actual activation by detecting and/or quantifying the phosphorylation level of a variety of different cellular targets of kinases/phosphatases, resulting from the equilibrium between these kinases/phosphatases as provided in US 2004/0265938.

The ratios of phosphorylated level of proteins can be compared to samples of cells being either not or sufficiently activated, so that the experimental values of the ratio provide an evaluation of the cell activation compared to the two extreme cell situations.

The same sample is preferably incubated with capture probes immobilized in two compartments, the first being used for the quantification of the total proteins captured and the second for the quantification of the phosphorylated proteins using two sets of recognition reagents, with the ratio between the two quantifications giving the level of activation of the cells. The two compartments may contain a plurality of identical capture probes for the target proteins to be detected, and the level of target proteins is quantified in one compartment using recognition reagents being specific for each of the proteins and the level of phosphorylation of target proteins is quantified in the other compartment using recognition reagents being specific for the phospho-proteins.

The method is particularly well suited for screening purposes as in this case, there is no indication of the possible proteins to be present. So the larger the number of capture probes on the array, the better the screening results.

In a particular application, the invention is used to screen proteins being bound to some DNA sequences and to identify and/or quantify the proteins. The DNA sequences are immobilized in the different compartments of the chamber and the samples possibly containing said proteins are incubated in order for the proteins to recognize the specific sequences of the DNA on which they will bind. The identification of the proteins is then performed by the use of specific recognition reagents preferably specific antibodies and the detection is performed as provided in the different embodiments of this invention. In a specific application, the same DNA sequence is present in more than one compartment and different specific recognition reagents are used in different compartments in order to detect different proteins bound to the same sequence. The method is well suited for the analysis and comparison of the pattern of proteins which are bound to different DNA sequences such as the promoters of genes. The presence of specific proteins or a pattern of proteins on the promoter sequence of a gene provides information on the regulation of its transcription.

In a specific application, the invention provides capture probes being single strand polynucleotides which are bound to specific locations of the compartments of the chamber wherein the polynucleotide of one compartment has an homologous sequence which differs by a single base from the polynucleotide present in another compartment and better has three homologous sequences which each one differing by a single base from the polynucleotide present in three other compartments. The invention is then able to discriminate between target polynucleotide sequences which differ by one base at the question sequence. The application is typically the Single Nucleotide Polymorphism (SNP) detection. In still another preferred embodiment, the four compartments contain capture polynucleotides which have all the same nucleotide at the question sequence being in one compartment Adenosine (A), in the second one Guanosine (G) in the third one Cytidine (C) and in the fourth one Thymidine (T) or Uridine (U). Such a configuration gives a particularly easy analysis of the SNP since one nucleotide is associated with one compartment.

In another application, the polynucleotide sequences to be detected are from the same gene but differ due to an alternative splicing. The capture probes are polynucleotides being in a single strand and being designed such as to bind at least in part to a sequence specific of the different forms of alternative splicing of a gene. The different sequences questioning the different alternative splicing variants are provided in the different compartments of the chamber and the detection is performed as described in the invention.

In a preferred embodiment, the invention provides a solid support for the detection and/or quantification of multiple different targets in a same sample, said support comprising: one or more chambers (1) comprising at least two compartments (2), and capture probes (4) being immobilized on a solid support surface (3) in (or of) the compartments, wherein the compartments (2) are in fluidic contact or are not in fluidic contact with each other according to the volume (v1, v2) of solution present in the chamber (1).

In a particular embodiment, one of the compartments (2) has a distinctive sign compared to the other compartments which is detectable together with the detection of the targets. In particular, the distinctive sign is a location bearing a particular signal or located in a particular part of the compartment in order to make this compartment distinctive of the other ones.

In a preferred embodiment, the at least two compartments (2) of the chamber (1) contain one or even more than 4 or even more than 10 identical capture probes (4).

In another embodiment, the at least two compartments (2) of the chamber (1) are fixed with different capture probes (4).

In a particular embodiment, the capture probes (4) are bound onto the solid support (3) in discrete regions in the form of an array having a density of at least 4 discrete regions per cm² and better 10 and even better 100 and even 1000. Particularly, the array comprises capture probes (4) which are specific of different targets.

In a specific embodiment, the chamber (1) is in the form of a well or in the form of a strip of 8 wells or a multiple of such strips such as a 96 wells plate. The chamber has preferably of circle or square form of between 0.4 and 0.6 cm² surface and a height of between about 4 and about 10 mm. The chamber is compartmentalized with 2, or more preferably 4, or even 8 or 16 compartments. The compartments are preferably separated by (possibly removable or openable) physical boundaries separations or walls (9) with height of between about 1 and about 3 mm. In a specific embodiment, the well compatible with a 96-wells format is 0.4 to 1 cm high and has 4 compartments separated by boundaries of 2 mm high. The volume (v1) of solution added in a compartment (2) is comprised between about 1 and about 20 µl (preferably 10 µl) and the volume (v2) of solution present in chamber (1) or well is comprised between about 10 and about 1000 µl (preferably about 150 µl). In another embodiment, the volume (v2) of solution present in the chamber (1) is between 1.5 and 10 times the volume (v1) of solution present in each compartment (2) multiplied by the number of compartments.

In a preferred embodiment, the solid support has a microtiter plate format and the chambers (1) are wells. Particularly, the microtiter plate has 96 wells (12 x 8) .

In still another embodiment, the wells are between 1.5 and 3 cm² of surface and comprise in least 2 and better 4 and still better 16 and still better 64 and even 384 or 1536 compartments (2). The compartments (2) are preferably separated by physical boundaries with height of between about 1 and about 3 mm.

In still another embodiment, the chamber is composed of different layers of material which cover the support (3) and which are delimitating a chamber (1). In a preferred embodiment, the number of layers around the chamber is different from the number of layer(s) inside the chamber thus delimitating compartments (2) in the chamber (1). In a particular embodiment, the support is covered with 2 layers of material (bottom and top layers) which are cut in order to delimitate the chamber and one is partly cut (bottom layer) so that it delimitates the compartments in the chamber. A drawing of such a device is presented in figure 3.

In a particular embodiment, the support is a glass slide of the microscopic format (2.5 ± 0.2 cm x 7.5 ± 0.2 cm). The solid support comprises at least 2 and better 4 and even better 8 different chambers (1) or wells obtained by removal of at least 2 layers at the place of the chambers. In each chamber, one layer is cut in order to provide empty compartments but leaving boundaries between the compartments.

In still another embodiment, the solid support is a microscopic slide and the chambers are 24 wells (3 x 8) .

In a preferred embodiment, compartments are obtained by placing short boundaries inside a well (figure 2). Boundaries in the form of (possibly removable or openable) physical separations (9) are positioned at the bottom of the well to divide the space into four compartments (2). The physical separations (9) are attached to the bottom of the chamber. The outer wall of the well extends upward between 5 and 10 mm from the bottom of the well and the height of the boundaries (physical separations) is between 1 and 3 mm. Thus the well can be overfilled to interconnect the four compartments in a fluidic contact.

In another embodiment, compartments (2) are obtained by forming depression or sub-wells inside a well. The outer wall of the well is higher than the depression walls (9) of the individual sub-wells or compartments. The height of the depression walls (9) is between 1 and 3 mm. The sub-wells are preferably configured in a regular array and are separated by approximately 0.02 cm. The device is configured to allow overfilling of each sub-well in order to interconnect them in a common fluid medium.

In an alternative embodiment, compartments (2) are obtained by placing small sub-wells inside a well. Sub-wells are positioned at the bottom of the main well. Each sub-well is defined by a bottom and a wall. The height of the sub-well walls (9) is between 1 and 3 mm. The device is configured to allow overfilling of each sub-well in order to interconnect them in a common fluid medium.
In a particular embodiment the separation or the sub-wells are removed after the incubation performed in individual compartment. This means that the fluidic contact between the compartments (2) is obtained by a removal of their physical separations (9) .

The invention can be applied to any multi-well format with any number of wells or chambers per plate of any shape (rectangular, square, round).

In an alternative embodiment, the physical separations are attached to the top of the chamber. They form compartments that can be filled individually. The compartments are then removed when necessary for incubation with the same solution on the overall chamber.

In still another alternative embodiment, the separations between the compartments are performed by the presence of different chemical components on the bottom of the chamber. The preferred chemical separation is obtained with hydrophobic compound designing the compartments.

### Examples

The following examples are provided for illustrative purpose only, and are not intended to limit the scope of the invention.

### Example 1. Background reduction upon compartmentalizing the assay

### A. Immobilization of capture probes on a solid support

Protein capture probes are spotted in triplicates on the surface of functionalized glass slides (Diaglass, EAT, Namur, Belgium) as follows: they are dissolved in a borate buffer supplemented with detergent and sugar molecules to preserve their activity and are spotted at a concentration of 1mg/ml under controlled conditions of humidity and temperature, using a pin and ring. The array design as well as the capture probes used are presented in figure 4.

After spotting, slides are washed, and a blocking is performed in PBS supplemented with dried milk for one hour at RT, followed by washing. Slides are dried and hybridization chambers are apposed onto the slides to physically separate the arrays and create compartments.

### B. Cell activation

Jurkat cells are grown in 75 cm² flasks in RPMI medium supplemented with 10% fetal bovine serum and antibiotics. They are stimulated with 50 ng/ml PMA for 5 min, and total protein extracts are prepared as described (Assay Designs, USA, using Assay Buffer 22 (#80-1114) supplemented with protease inhibitors)

### C. Sample incubation and detection

Fifty µg of protein extract are diluted in an incubation buffer (Assay buffer 4, Assay Designs, USA) supplemented with protease inhibitors and are contacted with four compartments of a slide. Binding is performed for 2 hours at RT under gentle agitation in a Thermomixer (Eppendorf). Compartments are then emptied and washed.

### D. Detection

Compartment 1 is contacted with a rabbit anti-phospho-MEK1/2 (Ser217/221) antibody (Cell Signaling Technology, USA; antibody #1); compartment 2 is contacted with a mix of antibody #1 and a rabbit anti-phospho-38 MAPK (T180/Y182) antibody (R&D Systems, USA; antibody #2); compartment 3 is contacted with a mix of a rabbit anti-phospho-c-RAF (Ser338) (56A6) antibody (Cell Signaling Technology, USA; antibody #3), a rabbit anti-phospho-MSK1 (S212) antibody (R&D Systems, USA; antibody #4) and a rabbit anti-phospho-ERK1/2 (pT185/pY187) antibody (Biomol, USA; antibody #5); compartment 4 is contacted with a mix of antibodies #1, #2, #3, #4 and #5. Incubation is performed for 1h at room temperature, followed by washing.

The hybridization chambers are removed, and a Cy3-labeled secondary antibody (goat-anti-rabbit-Cy3, Jackson ImmunoResearch, USA) is contacted with the arrays for 1h at room temperature, followed by washing. Slides are then dried, and fluorescence detection is performed by scanning the slide using a laser confocal scanner "ScanArray" (Packard, USA) at a resolution of 10 µm and a PMT setting of 80.

Results are summarized hereafter. The background measured on the support from compartments 1 to 4 is respectively of 1686 (1 antibody), 2963 (2 antibodies), 8188 (3 antibodies) and 11008 A.U. (5 antibodies). We observe that the background dramatically increases with the number of antibodies mixed at the detection step. For those antibodies which generate a high background, compartmentalizing the detection step with a maximum of two antibodies per compartment allows to reduce the background by a factor which can be as high as 3.

### Example 2. Simultaneous detection of proteins with structural similarities

Steps A, B and C are performed as in the example above.

### D. Detection

Compartment 1 is contacted with antibody #2, compartment 2 is contacted with antibody #5 and compartment 3 is contacted with a mix of antibodies #2 and #5. Incubation is performed for 1h at room temperature, followed by washing.

The hybridization chambers are removed, and a Cy3-labeled secondary antibody (goat-anti-rabbit-Cy3, Jackson ImmunoResearch, USA) is contacted with the arrays for 1h at room temperature, followed by washing. Slides are dried, and fluorescence detection is performed by scanning the slides using a laser confocal scanner "ScanArray" (Packard, USA) at a resolution of 10 µm and a PMT setting of 100.

Results are presented in figure 5. We observe that the anti-phospho-ERK1/2 antibody gives a specific signal on its capture probe (ERK1/2 probe, compartment 2), while the anti-phospho-p38 antibody gives a specific signal for p38 [p38 (1) and (2) probes, compartment 1] and cross-reacts with ERK1/2 (ERK1/2 probe, compartment 1). Combining the two antibodies at the detection step (p38 + ERK1/2, compartment 3) shows that it is impossible to determine the contribution of both antibodies for the ERK1/2 signal, which is generated by the specific anti-ERK2 detection and by the cross-reaction with the anti-p38 detection. The lack of specificity of the anti-phospho-p38 antibody claims for a compartmentalization assay, where the capture probes of ERK1/2 and p38 are spotted in different compartments, and where each compartment is contacted with its respective detection antibodies.

### Example 3. Analysis of transcriptional factors on microarray upon activation

### A. Cellular activation

Hela cells are grown in 75 cm² flasks DMEM medium, high glucose, supplemented with 10% fetal bovine serum and antibiotics. They are stimulated at 8 x 10⁶ cells/75cm² flasks with 5µM anisomycin for 1h. The nuclear extracts are prepared as described in WO 01/73115.

### B. Transcription factor (TF) microarrays

The TFChips MAPK kit was provided by Eppendorf (Hamburg, Germany). Transcription factor microarrays are present on glass slides. The arrays contain as capture probes double-stranded DNA for the TF binding. Each transcription factor microarray is composed of triplicate spots allowing the assay of AP1, STAT1, ELK1, p53, c-MYC, ATF2, NFAT and MEF2.

### C. Detection of activated transcription factors on TFChip MAPK.

A blocking step is performed in a Blocking Solution (TFChip MAPK kit, Eppendorf, Germany) for 1h at RT, followed by washing with TF Washing Solution (TFChip MAPK kit, Eppendorf, Germany). The nuclear extracts from the samples to be analyzed are diluted in a Sample Solution and Binding Solution (TFChip MAPK kit, Eppendorf, Germany) and contacted with the arrays. Incubation is performed overnight at room temperature and under agitation in a thermomixer (Eppendorf, Germany). Samples are removed and microarrays are washed 3 times with TF Washing Solution (TFChip MAPK kit, Eppendorf, Germany). Slides are dried and chambers are apposed onto the slides to physically separate the arrays and create compartments.

Compartment 1 is contacted with a rabbit anti-ATF2 antibody (Santa Cruz Biotechnology, USA; antibody #6), compartment 2 is contacted with a mouse anti-phospho-ATF2 antibody (Santa Cruz Biotechnology; antibody #7), and compartment 3 with a mix of antibodies #6 and 7. Antibodies are diluted 1000x in a Blocking Solution (TFChip MAPK kit, Eppendorf, Germany). Incubation is performed for 1h at RT, followed by washing 3 times with TF Washing Solution (TFChip MAPK kit, Eppendorf, Germany).

Chambers are removed, and microarrays are contacted with Cy3-labeled secondary antibodies under indirect light for 45 min at RT, followed by washing 3 times with TF Washing Solution and once with Washing Buffer B (TFChip MAPK kit, Eppendorf, Germany).

Slides are dried. A fluorescence detection of signals is performed by scanning slides using a laser confocal scanner "ScanArray" (Packard, USA) at a resolution of 10 µm and a gain of 80. Signals are analyzed using the Eppendorf Data Analysis Software for TFChip.

Figure 6 shows the results obtained. When combining the two detection antibodies (ATF2 + p-ATF2, compartment 3) directed against the non-phosphorylated (ATF2) and phosphorylated (p-ATF2) forms of ATF2, a signal is measured which results from the individual signals generated by the antibodies. The contribution of each antibody (ATF2, compartment 1) and (p-ATF2, compartment 2) cannot be assessed using this total signal, however, as both antibodies compete to bind the same protein target and might lead to some interference. It therefore necessitates performing the detection step in individual compartments.

### Example 4: Simultaneous detection of the monomers of the AP1 transcriptional factor

### A. Cellular activation

Hela cells are grown in 75 cm² flasks DMEM medium, high glucose, supplemented with 10% fetal bovine serum and antibiotics. They are stimulated at 8 x 10⁶ cells/75cm² flasks with 0.1 µg/ml PMA + 1 µM ionomycin for 1h. The nuclear extracts are prepared as described in WO 01/73115.

### B. Transcription factor (TF) microarrays

The TFChips MAPK kit was provided by Eppendorf (Hamburg, Germany). Transcription factor microarrays are present on glass slides. The arrays contain as capture probes double-stranded DNA for the TF binding. Each transcription factor microarray is composed of triplicate spots allowing the assay of AP1, STAT1, ELK1, p53, c-MYC, ATF2, NFAT and MEF2.

### C. Detection of monomers constituting the AP1 dimer on microarray.

A blocking step is performed in a Blocking Solution (TFChip MAPK kit, Eppendorf, Germany) for 1h at RT, followed by washing with TF Washing Solution (TFChip MAPK kit, Eppendorf, Germany). The nuclear extracts from the samples to be analyzed are diluted in a Sample Solution and Binding Solution (TFChip MAPK kit, Eppendorf, Germany) and contacted with the arrays. Incubation is performed overnight at room temperature and under agitation in a thermomixer (Eppendorf, Germany). Samples are removed and microarrays are washed 3 times with TF Washing Solution (TFChip MAPK kit, Eppendorf, Germany). Slides are dried and chambers are apposed onto the slides to physically separate the arrays and create compartments.

Compartment 1 is contacted with a rabbit antic-Fos antibody (Santa Cruz Biotechnology, USA; antibody #8), compartment 2 is contacted with a mouse anti-phospho-c-Jun antibody (Cell Signaling Technology; antibody #9), and compartment 3 with a mix of antibodies #8 and 9. Antibodies are diluted 1000x in a Blocking Solution (TFChip MAPK kit, Eppendorf, Germany). Incubation is performed for 1h at RT, followed by washing 3 times with TF Washing Solution (TFChip MAPK kit, Eppendorf, Germany).

Chambers are removed, and microarrays are contacted with Cy3-labeled secondary antibodies under indirect light for 45 min at RT, followed by washing 3 times with TF Washing Solution and once with Washing Buffer B (TFChip MAPK kit, Eppendorf, Germany).

Slides are dried. A fluorescence detection of signals is performed by scanning slides using a laser confocal scanner "ScanArray" (Packard, USA) at a resolution of 10 µm and a PMT setting of 80. Signals are analyzed using the Eppendorf Data Analysis Software for TFChip.

Figure 7 shows that the AP1 complex activated in Hela + PMA/ionomycin contains c-Fos and c-Jun monomers. In addition to the AP1 signal, a signal appears on the ATF2 spots, due to the well known similarities in the specific DNA binding sites of the two transcriptional factors. When combining the two detection antibodies (c-Fos + p-c-jun, compartment 3) specific of the monomers, a signal is measured which results from the individual signals generated by the separate antibodies. The contribution of each antibody (c-Fos, compartment 1) and (p-c-Jun, compartment 2) cannot be determined from this total signal, however, as differences in signal intensities observed for different transcriptional factors, or transcriptional factor monomers, do not necessarily reflect differences in transcriptional factor concentration. Each signal indeed depends on many parameters among which the affinity of the corresponding antibody.

## Claims

1. A method for a detection and/or quantification of multiple and different targets in a sample, comprising the steps of :
- Incubating a sample solution containing the targets into a chamber (1) comprising at least two compartments (2), in the presence of capture probes (4) specific of the targets and being immobilized on a solid support (3) in the compartments (2) (step 1) ;
- performing a detection and/or quantification of the targets bound to the capture probes by successive additions of solutions (v1, v2) containing reagents (step 2, 3, 4) and/or washing solutions (step 1', 2', 3', 4') into the chamber and/or in the compartments;
wherein in at least one step (step 2), a solution (v1) of recognition reagents (5) specific of a first group of bound targets are incubated in a first compartment of the chamber and a second solution of recognition reagents (6) specific of a second group of bound targets are incubated in a second compartment of the chamber, being not in fluidic contact with the first compartment of the same chamber, and wherein in at least another step (step 1, 3, 4) a solution (v2) is added to the chamber allowing a fluidic contact with all the compartments of the chamber.

2. The method according to claim 1, wherein the compartments (2) are in fluidic contact or are not in fluidic contact with each other according to the volume (v1, v2) of solution added in the chamber (1).

3. The method according to claim 1 or 2, wherein the volume (v2) of solution added in the chamber (1) is between 1.5 and 10 times the volume (v1) of solution added in each compartment (2) multiplied by the number of compartments.

4. The method according to any of the claims 1 to 3, wherein the volume (v1) of solution added in a compartment (2) is comprised between 1 and 20 µl and the volume (v2) of solution added in a chamber (1) is comprised between 10 and 1000 µl.

5. The method according to any of the claims 1 to 4, wherein the steps (step 1, 3, 4) comprising addition of a volume (v2) of solution to the chamber (1) with fluidic contact between all the compartments (2) is a step selected from the group consisting of:
- incubation of a solution containing the targets with the capture probes (step 1),
- incubation with an universal reagent (7) being specific of the recognition reagents (5, 6) (step 3),
- incubation with labeling reagent (8) being specific of the said universal reagent (7) (step 4).
- One or more washings steps (step 1', 2', 3' and/or 4') of the chamber.

6. The method according to any of the claims 1 to 5, wherein the targets are selected from the group consisting of: nucleotide sequences, proteins, peptides, polysaccharides or a mixture thereof.

7. The method according to any of the claims 1 to 6, wherein the capture probes (4) are selected from the group consisting of: polynucleotide sequences, proteins, peptides, antibodies or fragments thereof, polysaccharides or a mixture thereof.

8. The method according to claim 7, wherein the polynucleotides capture probes (4) present in different compartments (2) allow a detection and/or quantification of targets differing by a single nucleotide.

9. The method according to claim 7, wherein the polynucleotide capture probes (4) present in different compartments (2) allow a detection and/or quantification of different alternative splicing variants.

10. The method according to any of the claims 1 to 9, wherein the targets are proteins and the capture probes (4) are polynucleotide sequences more particularly aptamers.

11. The method according to any of the claims 1 to 10, wherein the targets are proteins being transcriptional factors.

12. The method according to claim 11, wherein the capture probes (4) contain double-stranded DNA sequences comprising specific sequences able to bind different transcriptional factors.

13. The method according to claim 11, wherein the method allows for the determination of activated transcriptional factors.

14. The method according to claim 13, wherein the activated transcriptional factors are selected from those listed in table 3.

15. The method according to any of the claims 1 to 14, wherein the targets are binding partners of a known protein being immobilized in different compartments (2).

16. The method according to any of the claims 1 to 15, wherein the recognition reagents (5, 6) are antibodies specific of different targets.

17. The method according to claims 1 to 16, wherein the at least two compartments (2) of the chamber (1) are fixed with different capture probes (4).

18. The method according to claims 1 to 16, wherein the at least two compartments (2) of the chamber (1) are fixed with the same capture probes (4).

19. The method according to claim 18, wherein the same capture probes (4) are bound by different targets to be detected and/or quantified.

20. The method according to any of the preceding claims, wherein the different targets to be detected and/or quantified in different compartments (2) are different members from the same family of transcriptional factors.

21. The method according to claim 20, wherein the transcriptional factor families are selected from those listed in table 1.

22. The method according to any of the preceding claims, wherein the specific recognition reagents (5, 6) recognize a specific monomer of the different targets to be detected and/or quantified being multimeric proteins.

23. The method according to any of the preceding claims, wherein the specific recognition reagents (5, 6) used in the different compartments (2) allow the identification of the monomers of the target being a multimeric protein.

24. The method according to the claims 22 to 23, wherein the transcriptional factors are selected from those listed in table 2.

25. The method according to any of the preceding claims, wherein the specific recognition reagents (5, 6) recognize a non phosphorylated form of a target in a first compartment and a phosphorylated form of the same target in a second compartment separated from the first compartment.

26. The method according to the claim 25, wherein the determination of phosphorylated to non phosphorylated target ratio provides the degree of activation of a kinase.

27. The method according to any of the preceding claims, wherein the recognition reagents (5, 6) carry a first member of a binding pair and the universal reagent (7) a second member of the binding pair.

28. The method according to claim 27, wherein the binding pair is selected from the group consisting of biotin/streptavidin, biotin/anti-biotin, hapten/receptor and antigen/antibody.

29. The method according to any of the preceding claims, wherein the universal reagent (7) is labeled.

30. The method according to claim 29, wherein the label is directly detected or is detected through the use of a labeling reagent (8).

31. The method according to claim 30, wherein the label is gold and the labeling reagent (8) is Ag+ and a reducing agent.

32. The method according to any of the preceding claims, wherein the capture probes (4) are bound onto the solid support (3) in discrete regions in the form of an array having a density of at least 4, preferably 10, 100 or 1000 discrete regions per cm².

33. The method according to claim 32, wherein the array comprises capture probes (4) which are specific of different targets.

34. The method according to any of the preceding claims, wherein the chamber (1) comprises at least 2, preferably 4, 16, 64, 384 or 1536 compartments (2).

35. The method according to any of the preceding claims used for the detection of at least 10, preferably 20, 100 or 1000 targets present in the same sample.

36. The method according to any of the preceding claims, which requires the use of at least 10, preferably 20, 100 or 1000 different recognition reagents (5, 6) .

37. The method according to any of the preceding claims, wherein the background level of the assay is reduced by a factor of 2 and better reduced by a factor of 4 in the compartmentalized assay compared to the non compartmentalized assay.

38. The method according to any of the preceding claims, wherein the fluidic contact between the compartments (2) is obtained by a removal of their physical separations (9).

39. A solid support for a detection and/or quantification of multiple and different targets in a sample, comprising:
one or more chambers (1) having at least two compartments (2), and capture probes (4) being specific of targets and immobilized on the surface of the said compartments, wherein the compartments (2) are in fluidic contact or are not in fluidic contact with each other according to the volume (v1, v2) of solution present in the chamber (1).

40. The solid support according to claim 39, wherein the chamber (1) comprises at least 2, preferably 4, 16, 64, 384 or 1536 compartments (2).

41. The solid support according to claim 39 to 40, wherein at least two compartments (2) of the chamber (1) contain one, preferably 4 or 10 identical capture probes (4).

42. The solid support according to any of the claims 39 to 41, wherein at least two compartments (2) of the chamber (1) are fixed with different capture probes (4).

43. The solid support according to any of the claims 39 to 42, wherein the capture probes (4) are bound onto the solid support (3) in discrete regions in the form of an array having a density of at least 4, preferably 10, 100 or 1000 discrete regions per cm².

44. The solid support according to claim 43, wherein the array comprises capture probes (4) which are specific of different targets.

45. The solid support according to any of the claims 39 to 44, which has a microtiter plate format and the chambers (1) are wells.

46. The solid support according to claim 45, wherein the microtiter plate has 96 wells (12 X 8) or 24 wells (6 X 4).

47. The solid support according to any of the claims 39 to 46, which is a microscopic slide and the chambers (1) are wells.

48. The solid support according to claim 47, wherein the microscopic slide has 24 wells (3 X 8).

49. The solid support according to any of the claims 39 to 48, wherein the chamber (1) comprises at least 2, preferably 4, 16, 64, 384 or 1536 compartments (2).

50. The solid support according to any of the claims 39 to 49, wherein one of the compartments (2) has a distinctive sign different from the distinctive sign of the other compartments which is detectable together with the detection of the targets.

51. The solid support according to any of the claims 39 to 50, wherein the compartment (2) are separated from each other by physical separations (9).

52. The solid support of claim 51, wherein the physical separations (9) have a height comprised between 1 and 3 mm, and wherein the chambers have a height comprised between 4 and 10 mm.

53. The solid support of claim 51, wherein the physical separations (9) are attached to the bottom of the chamber

54. The solid support of claim 51, wherein the physical separations (9) are attached to the top of the chamber.

55. The solid support of claim 51, wherein the physical separations (9) are removal parts.

56. The solid support to any of the claim 39 to 51, wherein the separations (9) between the compartments are performed by the presence of different chemical components present on the bottom of the chamber (1).

57. A kit for a detection and/or quantification of multiple different targets in a sample, which comprises:
- the solid support of claims 39 to 56;
- recognition reagents (5, 6) being primary antibodies or specific hypervariable portions thereof, being specific of the different targets;
- optionally a universal reagent (7) being (a) secondary labeled antibody (ies) directed against all the recognition reagents (5, 6), and
- optionally a labeling reagent (8) being a specific substrate(s) of the universal reagent (7).

58. A kit of parts comprising the solid support of claims 39 to 56,
- a detection and quantification device of a signal formed at the location of the binding between a target and a capture probe on the solid support surface,
- possibly a reading device of information recorded on a surface of the solid support,
- a computer program for recognizing the discrete regions bearing the bound targets upon corresponding capture probes and its locations on the solid support surface,
- possibly a quantification program of the signal present at the locations and a program for correlating the presence of the signal at these locations with the diagnostic and the quantification of the target to be detected and assigning the locations of the target according to the compartment (2) to which it belongs.
